# EUROPEAN PATENT APPLICATION

(11) **EP 4 789 613 A1**
(43) Date of publication of application: **12.08.2026**
(21) Application number: 26156449.6
(22) Date of filing: 04.02.2026
(51) Int. Cl.: A61B 6/03, A61B 6/40, A61B 6/51, A61B 6/00

(54) **GENERATING A PANORAMIC RADIOGRAPHY IMAGE**

(30) Priority: 05.02.2025 US 202519046251
(71) Applicant: Zetta25 AG, 8047 Zurich (CH)
(72) Inventor: EICHNER, Stefan, 69123 Heidelberg (DE)
(74) Representative: Taor, Simon Edward William

(57) **Abstract**

A method is provided for generating a panoramic radiography image of a body part of a patient with reduced background interference. The method includes capturing multiple radiography images of the body part along a predetermined panoramic trajectory using an imaging device, retrieving a 3D model of the body part from at least one data source, forming a panoramic radiography image from the captured radiography images, and registering the 3D model with that panoramic image. The method then identifies one or more interference areas in the 3D model, creates virtual panoramic projection images of the identified interference areas, and utilizing the virtual panoramic projection images, the method removes the identified interference areas from the individual radiography images or the panoramic radiography image. The resulting panoramic radiography image with reduced or no interference is presented to a medical professional for faster, more accurate diagnosis.

## Description

### FIELD

The present disclosure generally relates to an imaging device and a method for generating a panoramic radiography image with reduced interference and more particularly to a method for removing background interference in an extraoral panoramic X-ray image captured using the imaging device.

### BACKGROUND

Radiography images are commonly used as a standard procedure for diagnosing various medical and dental conditions. Different types of imaging devices are used for diagnosis, including extraoral X-ray imaging devices that capture panoramic radiography or X-ray images of a body part of a patient. For diagnosis of dental conditions, the panoramic X-ray images provide a comprehensive view of the jaws of the patient including the upper and the lower jaws, the temporomandibular joint, and surrounding structures. In practice, an extraoral X-ray imaging device can acquire multiple two-dimensional (2D) X-ray images at different angles by moving along a trajectory around the body part such as head of the patient. These 2D X-ray images can then be combined to reconstruct a final panoramic X-ray image.

### SUMMARY

According to an embodiment of the present disclosure, a method is provided for generating a panoramic radiography image of a body part of a patient with reduced background interference or overlays. The method comprises capturing a plurality of radiography images of the body part along a predetermined panoramic trajectory using an imaging device, retrieving at least one three-dimensional (3D) model of the body part from at least one data source, forming a panoramic radiography image of the body part from the plurality of radiography images, and registering the 3D model with the panoramic radiography image so that both share substantially identical geometry and orientation. The method further includes identifying one or more interference areas in the 3D model that cause interference in the panoramic radiography image, generating a plurality of virtual panoramic projection images for these interference areas based on the same geometry used to form the panoramic radiography image, and removing the identified interference areas from the plurality of radiography images or the panoramic radiography image using the virtual panoramic projection images. The panoramic radiography image, free of the interference areas, can then be presented to a medical professional, such as a doctor, for faster and more accurate diagnosis.

In an embodiment, the method of retrieving the 3D model from the at least one data source includes receiving cone beam computed tomography (CBCT) data of the body part, identifying CBCT data that shares substantially the same geometry and orientation as the panoramic radiography image, and generating the 3D model from the identified CBCT data using an image processing method such as segmentation, mesh decimation etc. The method helps to obtain detailed volumetric information about the body part, which may not be captured by conventional two-dimensional radiography.

In an embodiment, the method of registering the 3D model with the panoramic radiography image involves aligning a plurality of anatomical features in the 3D model with corresponding features in the panoramic radiography image, allowing precise correlation between each 3D pixel or voxel in the 3D model and each 2D pixel in the captured panoramic radiography image.

In an embodiment, the method of identifying the one or more interference areas further includes detecting at least one of low-frequency artifacts from opposing jaw structures or background shadows from a spinal column or other anatomical features. This is advantageous because it allows clinicians to obtain a panoramic radiography image free of interference, providing a clearer view of anatomical structures such as teeth, cavities or jaw lines.

In an embodiment, the method of removing the one or more interference areas from the plurality of radiography images or the panoramic radiography image further includes projecting these interference areas from the 3D model into virtual panoramic projection images, modifying each radiography image to eliminate the interference, and reconstructing the panoramic radiography image from the modified radiography images.

In an alternate embodiment, the method of removing the one or more interference areas comprises projecting the one or more interference areas from the 3D model into the virtual panoramic projection images, generating a separate panoramic radiography image of the identified interference areas, and removing those interference areas from the panoramic radiography image based on the separate panoramic radiography image.

In another aspect of the invention, an imaging device is disclosed. The imaging device includes a processor, a storage unit configured to receive and store a plurality of radiography images captured along the predetermined panoramic trajectory, and a memory in communication with the processor. The memory stores one or more computer program instructions that, when executed by the processor, enable the imaging device to perform the above method steps to generate one or more radiography images or a panoramic radiography image without the interference areas, by comparing with the 3D model of the body part retrieved from at least one data source.

In an embodiment, the imaging device is an extraoral X-ray device. The extraoral X-ray device advantageously provides extraoral panoramic X-ray images of patients with minimal or no interference caused by opposing jaw areas or anatomical overlays such as the spinal column.

Embodiments described below include a non-transitory computer-readable storage medium comprising computer-executable instructions that, responsive to execution by a processor, cause a system or the imaging device to perform any of the described methods.

Embodiments described below also include a system with means for generating a panoramic radiography image with reduced interference.

### BRIEF DESCRIPTION OF THE DRAWINGS

Apparatuses and techniques for generating a panoramic radiography image with reduced interference are described with reference to the following drawings. The same numbers are used throughout the drawings to reference like features and components: The drawings are of illustrative embodiments. They do not illustrate all embodiments. Other embodiments may be used in addition or instead. Details that may be apparent or unnecessary may be omitted to save space or for more effective illustration. Some embodiments may be practiced with additional components or steps and/or without all the components or steps that are illustrated. When the same numeral appears in different drawings, it refers to the same or like components or steps.
FIG. 1 depicts a block diagram of a data processing environment in which illustrative embodiments may be implemented.
FIG. 2 depicts a block diagram of a data processing system in which illustrative embodiments may be implemented.
FIG. 3 is a flowchart of a method for generating a panoramic radiography image of a body part of a patient with reduced background interference.
FIG. 4A depicts an imaging device at a position A and is configured to move along a predefined trajectory of the x-ray source to capture the radiography images without interference caused by opposing jaw areas.
FIG. 4B depicts the imaging device moved along the predefined trajectory PT-PT' to position B to capture the radiography images with interference caused by opposing jaw areas.
FIG. 4C depicts the imaging device moved along the predefined trajectory PT-PT' to position C to capture the radiography images with interference caused by opposing jaw areas.
FIG. 4D depicts the imaging device moved along the predefined trajectory PT-PT' to position D to capture the radiography images without interference caused by opposing jaw areas.
FIG. 4E depicts the imaging device moved along the predefined trajectory PT-PT' to position E to capture the radiography images with interference caused by the spinal column.
FIG. 4F depicts the imaging device moved along the predefined trajectory PT-PT' to position F to capture the radiography images without interference caused by opposing jaw areas or the spinal column.
FIG. 5 depicts an example radiography image with interference caused by the spinal column and the opposing jaws obtained using the imaging device using conventional method.
FIG. 6 depicts an example 3D model or 3D slice used in generating a 3D model.
FIG. 7 depicts a 3D mesh used for generating a panoramic image of a dental area of the patient.
FIG. 8 depicts mapping of the 3D model or 3D mesh with the 2D panoramic radiography image captured using the imaging device.
FIG. 9 depicts a plurality of virtual panoramic projection images according to an illustrative embodiment.
FIG. 10 depicts an example reconstructed radiography image with less interference caused by the spinal column and the opposing jaws.

### DETAILED DESCRIPTION

### Overview

In the following detailed description, numerous specific details are set forth by way of examples to provide a thorough understanding of the relevant teachings. However, it should be apparent that the present teachings may be practiced without such details. In other instances, well-known methods, procedures, components, and/or circuitry have been described at a relatively high level, without detail, to avoid unnecessarily obscuring aspects of the present teachings.

Three-dimensional (3D) imaging or volumetric imaging is employed in modern diagnostics to capture detailed anatomical information about a specific body part of a patient. The anatomical information of the body part obtained from the 3D image can be utilized in assisting or improving various diagnostics including dental diagnostics, which typically use intra oral radiography images such as X-rays to assess teeth, jaw, and surrounding anatomy of a patient. However, this type of imaging only provides close-up views of individual or small groups of teeth of the patient. To overcome the drawbacks of the intraoral radiography images, extraoral panoramic radiography imaging is utilized by dentists in which an X-ray source and a corresponding detector, which forms part of an imaging device, rotate around a head of the patient along a trajectory to capture a broad view of the upper and lower jaws, including portions of the temporomandibular joint and adjacent regions.

It is recognized that the imaging device can capture multiple radiography or X-ray images at specific times while moving along the trajectory and then combine the images to form a single panoramic radiography or X-ray image, which provides a comprehensive overview of dental and maxillofacial regions. However, traditional panoramic radiography images may contain unwanted overlapping features or interference areas caused by spinal column shadows or opposing jaw structures, which make diagnosis difficult. It is further recognized that techniques utilizing tomographic reconstruction or utilizing cone beam computed tomography (CBCT) data can refine specific areas of the panoramic radiography images partially or using computational post-processing but may be unable to remove unwanted background interference without degrading the diagnostic quality of the panoramic radiography image.

Techniques are described herein that implement an imaging device and associated method for generating a panoramic radiography image with reduced background interference or overlays. In example aspects, a method is provided for generating a panoramic radiography image of a body part of a patient with reduced background interference or overlays. The method comprises capturing a plurality of radiography images of the body part along a predetermined panoramic trajectory using an imaging device, retrieving at least one three-dimensional (3D) model of the body part from at least one data source, forming a panoramic radiography image of the body part from the plurality of radiography images, and registering the 3D model with the panoramic radiography image so that both share the same or substantially the same geometry and orientation. The method further includes identifying one or more interference areas in the 3D model that cause interference in the panoramic radiography image, generating a plurality of virtual panoramic projection images for these interference areas based on the same geometry, orientation, and frame rate used to form the panoramic radiography image, and removing the identified interference areas from the plurality of radiography images or the panoramic radiography image using the virtual panoramic projection images. The panoramic radiography image, free of the interference areas, can then be presented to a medical professional, such as a doctor, for faster and more accurate diagnosis.

The illustrative embodiments are described with respect to certain types of machines. The illustrative embodiments are also described with respect to other scenes, subjects, measurements, devices, data processing systems, environments, components, and applications only as examples. Any specific manifestations of these and other similar artifacts are not intended to be limiting to the disclosure. Any suitable manifestation of these and other similar artifacts can be selected within the scope of the illustrative embodiments.

Furthermore, the illustrative embodiments may be implemented with respect to any type of data, data source, or access to a data source over a data network. Any type of data storage device may provide the data to an embodiment of the disclosure, either locally at a data processing system or over a data network, within the scope of the disclosure. Where an embodiment is described using a mobile device, any type of data storage device suitable for use with the mobile device may provide the data to such embodiment, either locally at the mobile device or over a data network, within the scope of the illustrative embodiments.

The illustrative embodiments are described using specific surveys, code, hardware, algorithms, designs, architectures, protocols, layouts, schematics, and tools only as examples and are not limiting to the illustrative embodiments. Furthermore, the illustrative embodiments are described in some instances using particular software, tools, and data processing environments only as an example for the clarity of the description. The illustrative embodiments may be used in conjunction with other comparable or similarly purposed structures, systems, applications, or architectures. For example, other comparable devices, structures, systems, applications, or architectures therefor, may be used in conjunction with such embodiment of the disclosure within the scope of the disclosure. An illustrative embodiment may be implemented in hardware, software, or a combination thereof.

The examples in this disclosure are used only for the clarity of the description and are not limiting to the illustrative embodiments. Additional data, operations, actions, tasks, activities, and manipulations will be conceivable from this disclosure and the same are contemplated within the scope of the illustrative embodiments.

Any advantages listed herein are only examples and are not intended to be limiting to the illustrative embodiments. Additional or different advantages may be realized by specific illustrative embodiments. Furthermore, a particular illustrative embodiment may have some, all, or none of the advantages listed above.

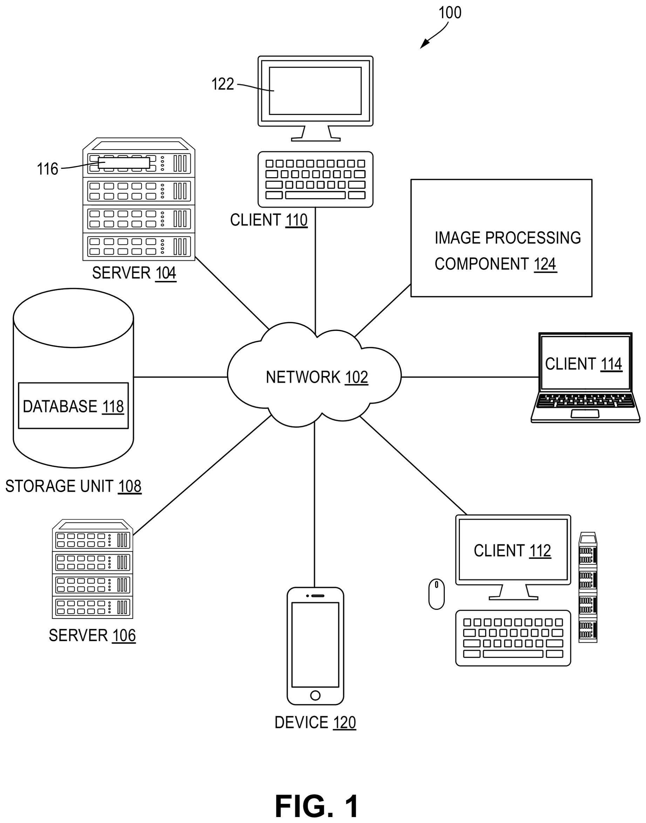

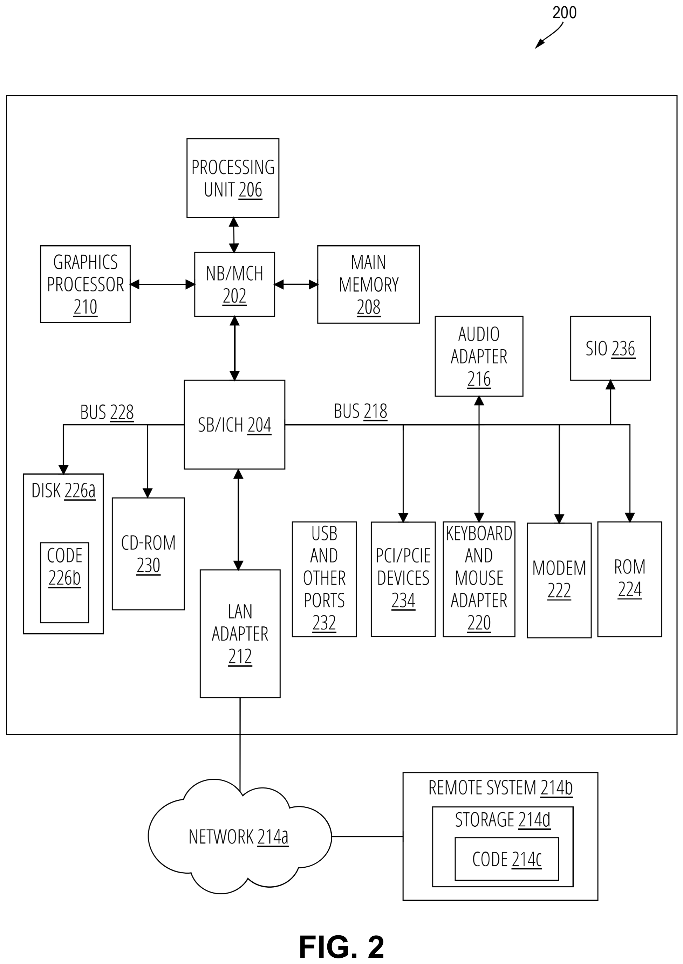

With reference to the figures and in particular, with reference to FIG. 1 and FIG. 2, these figures are example diagrams of data processing environments in which illustrative embodiments may be implemented. FIG. 1 and FIG. 2 are only examples and are not intended to assert or imply any limitation with regard to the environments in which different embodiments may be implemented. A particular implementation may make many modifications to the depicted environments based on the following description.

FIG. 1 depicts a block diagram of a network of data processing systems in which illustrative embodiments may be implemented. Data processing environment 100 is a network of computers in which the illustrative embodiments may be implemented. Data processing environment 100 includes network 102. Network 102 is the medium used to provide communications links between various devices and computers connected together within the data processing environment 100. Network 102 may include connections, such as wire, wireless communication links, or fiber optic cables.

Clients or servers are only example roles of certain data processing systems connected to network 102 and are not intended to exclude other configurations or roles for these data processing systems. Server 104 and server 106 couple to network 102 along with storage unit 108. Software applications may execute on any computer in data processing environment 100. Client 110, client 112, and client 114 are also coupled to network 102. A data processing system, such as server 104 or server 106, or clients (client 110, client 112, client 114) may contain data and may have software applications or software tools executing thereon. Server 104 may include one or more GPUs (graphics processing units) for training one or more models.

Only as an example, and without implying any limitation to such architecture, FIG. 1 depicts certain components that are usable in an example implementation of an embodiment. For example, servers and clients are only examples and not to imply a limitation to a client-server architecture. As another example, an embodiment can be distributed across several data processing systems and a data network as shown, whereas another embodiment can be implemented on a single data processing system within the scope of the illustrative embodiments. Data processing systems (server 104, server 106, client 110, client 112, client 114) also represent example nodes in a cluster, partitions, and other configurations suitable for implementing an embodiment.

Device 120 is an example of a device described herein. For example, device 120 can take the form of a smartphone, a special purpose fabrication platform, a tablet computer, a laptop computer, client 110 in a stationary or a portable form, a wearable computing device, or any other suitable device. Any software application described as executing in another data processing system in FIG. 1 can be configured to execute in device 120 in a similar manner. Any data or information stored or produced in another data processing system in FIG. 1 can be configured to be stored or produced in device 120 in a similar manner.

An image processing component 124 of an X-ray or imaging device may execute as part of client application 122, server application 116, or on any data processing system herein. The image processing component 124 may also execute as a cloud service communicatively coupled to system services, hardware resources, or software elements described herein. Database 118 of storage unit or data source 108 stores one or more data in repositories for computations herein. The image processing component 124 may perform the method comprising: capturing a plurality of radiography images of the body part along a predetermined panoramic trajectory using an imaging device, retrieving at least one three-dimensional (3D) model of the body part from at least one data source 108 or server 104 or 106, forming a panoramic radiography image of the body part from the plurality of radiography images, and registering the 3D model with the panoramic radiography image so that both share substantially identical geometry and orientation. The method further includes identifying one or more interference areas in the 3D model that cause interference in the panoramic radiography image, generating a plurality of virtual panoramic projection images for these interference areas based on the same geometry, orientation, and frame rate used to form the panoramic radiography image, and removing the identified interference areas from the plurality of radiography images or the panoramic radiography image using the virtual panoramic projection images. The panoramic radiography image, free of the interference areas, can then be presented to a medical professional, such as a doctor, through an interface of the client devices 110, 112 or 114 for faster and more accurate diagnosis.

Server application 116 implements an embodiment described herein. Server application 116 can use data from storage unit 108 for generating a panoramic radiography image with reduced interference. Server application 116 can also obtain data from any client for computations. Server application 116 can also execute in any of data processing systems (server 104 or server 106, client 110, client 112, client 114), such as client application 122 in client 110, and need not execute in the same system as server 104.

Server 104, server 106, storage unit 108, client 110, client 112, client 114, and device 120 may couple to network 102 using wired connections, wireless communication protocols, or other suitable data connectivity. Client 110, client 112, and client 114 may be, for example, personal computers or network computers.

In the depicted example, server 104 may provide data, such as boot files, operating system images, and applications to client 110, client 112, and client 114. Client 110, client 112, and client 114 may be clients to server 104 in this example. Client 110, client 112, and client 114 or some combination thereof, may include their own data, boot files, operating system images, and applications. Data processing environment 100 may include additional servers, clients, and other devices that are not shown. Server 104 includes a server application 116 that may be configured to implement one or more of the functions described herein in accordance with one or more embodiments.

The data processing environment 100 may also be the Internet. Network 102 may represent a collection of networks and gateways that use the Transmission Control Protocol/Internet Protocol (TCP/IP) and other protocols to communicate with one another. At the heart of the Internet is a backbone of data communication links between major nodes or host computers, including thousands of commercial, governmental, educational, and other computer systems that route data and messages. Of course, data processing environment 100 also may be implemented as a number of different types of networks, such as for example, an intranet, a local area network (LAN), or a wide area network (WAN). FIG. 1 is intended as an example, and not as an architectural limitation for the different illustrative embodiments.

Among other uses, data processing environment 100 may be used for implementing a client-server environment in which the illustrative embodiments may be implemented. A client-server environment enables software applications and data to be distributed across a network such that an application functions by using the interactivity between a client data processing system and a server data processing system. Data processing environment 100 may also employ a service-oriented architecture where interoperable software components distributed across a network may be packaged together as coherent business applications. Data processing environment 100 may also take the form of a cloud and employ a cloud computing model of service delivery for enabling convenient, on-demand network access to a shared pool of configurable computing resources (e.g., networks, network bandwidth, servers, processing, memory, storage, applications, virtual machines, and services) that can be rapidly provisioned and released with minimal management effort or interaction with a provider of the service.

With reference to FIG. 2, this figure depicts a block diagram of a data processing system in which illustrative embodiments may be implemented. Data processing system 200 is an example of a computer, such as server 104, server 106, or client 110, client 112, client 114, or image processing component 124 in FIG. 1, or another type of device in which computer-usable program code or instructions implementing the processes may be located for the illustrative embodiments.

Data processing system 200 is also representative of a data processing system or a configuration therein, such as device 120 in FIG. 1 in which computer-usable program code or instructions implementing the processes of the illustrative embodiments may be located. Data processing system 200 is described as a computer only as an example, without being limited thereto. Implementations in the form of other devices, such as device 120 in FIG. 1, may modify data processing system 200, such as by adding a touch interface, and even eliminate certain depicted components from data processing system 200 without departing from the general description of the operations and functions of data processing system 200 described herein.

In the depicted example, data processing system 200 employs a hub architecture including North Bridge and memory controller hub (NB/MCH) 202 and South Bridge and input/output (I/O) controller hub (SB/ICH) 204. Processing unit 206, main memory 208, and graphics processor 210 are coupled to North Bridge and memory controller hub (NB/MCH) 202. Processing unit 206 may contain one or more processors and may be implemented using one or more heterogeneous processor systems. Processing unit 206 may be a multi-core processor. Graphics processor 210 may be coupled to North Bridge and memory controller hub (NB/MCH) 202 through an accelerated graphics port (AGP) in certain implementations.

In the depicted example, local area network (LAN) adapter 212 is coupled to South Bridge and input/output (I/O) controller hub (SB/ICH) 204. Audio adapter 216, keyboard and mouse adapter 220, modem 222, read only memory (ROM) 224, universal serial bus (USB) and other ports 232, and PCI/PCIe devices 234 are coupled to South Bridge and input/output (I/O) controller hub (SB/ICH) 204 through bus 218. Hard disk drive (HDD) or solid-state drive (SSD) 226a and CD-ROM 230 are coupled to South Bridge and input/output (I/O) controller hub (SB/ICH) 204 through bus 228. PCI/PCIe devices 234 may include, for example, Ethernet adapters, add-in cards, and PC cards for notebook computers. PCI uses a card bus controller, while PCIe does not. Read only memory (ROM) 224 may be, for example, a flash binary input/output system (BIOS). Hard disk drive (HDD) or solid-state drive (SSD) 226a and CD-ROM 230 may use, for example, an integrated drive electronics (IDE), serial advanced technology attachment (SATA) interface, or variants such as external-SATA (eSATA) and micro- SATA (mSATA). A super I/O (SIO) device 236 may be coupled to South Bridge and input/output (I/O) controller hub (SB/ICH) 204 through bus 218.

Memories, such as main memory 208, read-only memory (ROM) 224, or flash memory (not shown), are some examples of computer usable storage devices. Hard disk drive (HDD) or solid-state drive (SSD) 226a, CD-ROM 230, and other similarly usable devices are some examples of computer usable storage devices including a computer usable storage medium.

An operating system runs on processing unit 206. The operating system coordinates and provides control of various components within data processing system 200 in FIG. 2. The operating system may be a commercially available operating system for any type of computing platform, including but not limited to server systems, personal computers, and mobile devices. An object-oriented or other type of programming system may operate in conjunction with the operating system and provide calls to the operating system from programs or applications executing on data processing system 200.

Instructions for the operating system, the object-oriented programming system, and applications or programs, such as server application 116 and client application 122 in FIG. 1, are located on storage devices, such as in the form of codes 226b on Hard disk drive (HDD) or solid-state drive (SSD) 226a, and may be loaded into at least one of one or more memories, such as main memory 208, for execution by processing unit 206. The processes of the illustrative embodiments may be performed by processing unit 206 using computer-implemented instructions, which may be located in a memory, such as, for example, main memory 208, read-only memory (ROM) 224, or in one or more peripheral devices.

Furthermore, in one case, code 226b may be downloaded over network 214a from remote system 214b, where similar code 214c is stored on a storage device 214d in another case, code 226b may be downloaded over network 214a to remote system 214b, where downloaded code 214c is stored on a storage device 214d.

The hardware in FIG. 1 and FIG. 2 may vary depending on the implementation. Other internal hardware or peripheral devices, such as flash memory, equivalent non-volatile memory, or optical disk drives and the like, may be used in addition to or in place of the hardware depicted in FIG. 1 and FIG. 2. In addition, the processes of the illustrative embodiments may be applied to a multiprocessor data processing system.

In some illustrative examples, data processing system 200 may be a personal digital assistant (PDA), which is generally configured with flash memory to provide non-volatile memory for storing operating system files and/or user-generated data. A bus system may comprise one or more buses, such as a system bus, an I/O bus, and a PCI bus. Of course, the bus system may be implemented using any type of communications fabric or architecture that provides for a transfer of data between different components or devices attached to the fabric or architecture.

A communications unit may include one or more devices used to transmit and receive data, such as a modem or a network adapter. A memory may be, for example, main memory 208 or a cache, such as the cache found in North Bridge and memory controller hub (NB/MCH) 202. A processing unit may include one or more processors or CPUs.

The depicted examples in FIG. 1 and FIG. 2 and above-described examples are not meant to imply architectural limitations. For example, data processing system 200 also may be a tablet computer, laptop computer, or telephone device in addition to taking the form of a mobile or wearable device.

Where a computer or data processing system is described as a virtual machine, a virtual device, or a virtual component, the virtual machine, virtual device, or the virtual component operates in the manner of data processing system 200 using virtualized manifestation of some or all components depicted in data processing system 200. For example, in a virtual machine, virtual device, or virtual component, processing unit 206 is manifested as a virtualized instance of all or some number of hardware processing units 206 available in a host data processing system, main memory 208 is manifested as a virtualized instance of all or some portion of main memory 208 that may be available in the host data processing system, and Hard disk drive (HDD) or solid-state drive (SSD) 226a is manifested as a virtualized instance of all or some portion of Hard disk drive (HDD) or solid-state drive (SSD) 226a that may be available in the host data processing system. The host data processing system in such cases is represented by data processing system 200.

Disclosed is a method for generating a panoramic radiography image with reduced interference to aid medical professionals, including doctors and technicians, in making more accurate and faster diagnoses. The panoramic radiography image, also referred to as an extraoral X-ray image, obtained using the present method offers a comprehensive, two-dimensional overview of the jaw, teeth, and surrounding anatomical structures of the patient, and is used in dental and maxillofacial diagnostics. The panoramic radiography image obtained without the background interference or overlays reduces overlapping regions that often appear in traditional panoramic images and provides a clear view of the dental and maxillofacial areas for planning complex procedures, including orthodontic corrections or dental implant placements. The panoramic radiography image also provides a wider view of the dental and maxillofacial areas in a single scan, thus reducing discomfort to the patients and helps to reduce the scanning time compared to multiple intraoral X-rays.

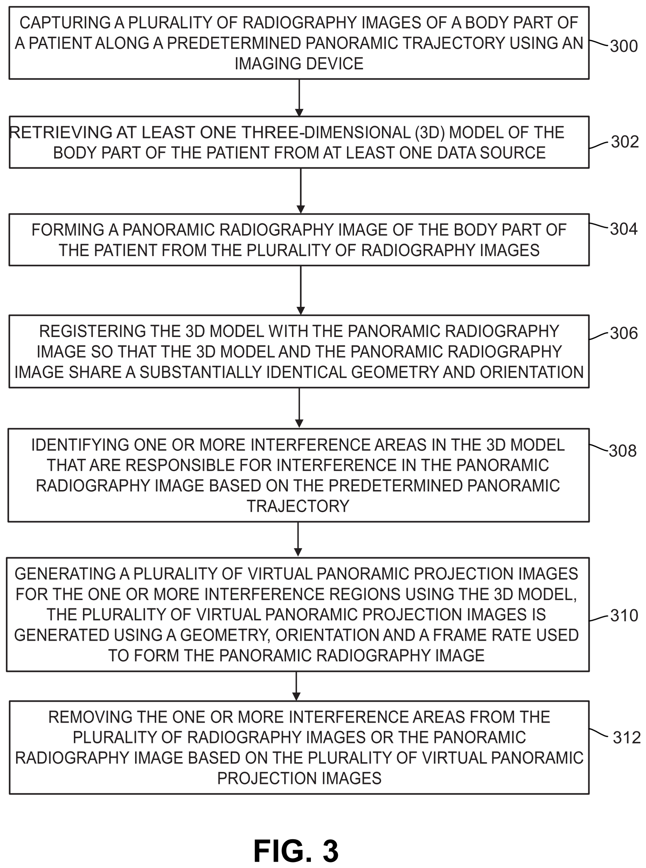

FIG. 3 depicts a flowchart of a method for generating a panoramic radiography image of a body part of a patient with reduced background interference. The method starts with block 300, capturing multiple radiography images or X-rays of a body part, such as the head of the patient, along a predetermined panoramic trajectory covering the dental area using an imaging device. In block 302, a three-dimensional (3D) model of the same body part of the same patient is retrieved from a data source. In block 304, a panoramic radiography image of the body part is formed from the captured radiography images. Next, in block 306, the method registers or aligns the 3D model with the formed panoramic radiography image or mesh so that both share substantially identical geometry and orientation in a common coordinate system. Then, in block 308, the method identifies one or more interference areas in the 3D model that cause interference in the panoramic radiography image. Once the interference areas, e.g., anatomical features or structures, causing the interference are identified, block 310 involves generating a plurality of virtual panoramic projection images by projecting the identified interference areas based on the same geometry, orientation, and frame rate used to form the panoramic radiography image. Finally, in block 312, the method removes or reduces the identified interference areas from the panoramic radiography image using a weighted subtraction technique. The extraoral panoramic image thus obtained has minimal background interference caused by anatomical features and can readily be used by doctors or medical practitioners to perform accurate diagnosis. Each block from 300 to 312 is explained in detail below with reference to the drawings.

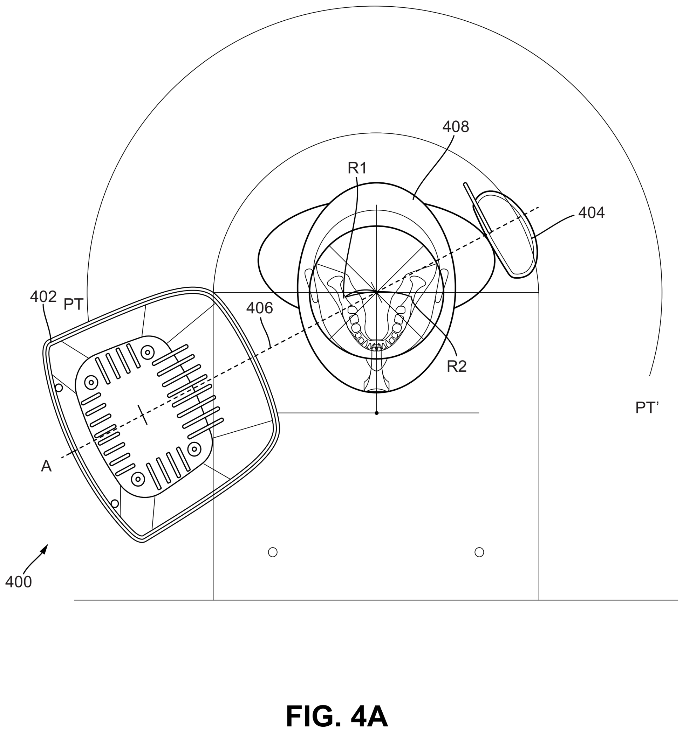

The block 300 of capturing multiple radiography images or X-rays of the body part such as head of the patient along the predetermined panoramic trajectory covering the dental area using the imaging device 400 is described by the drawings in FIGS. 4A - 4F. Initially, the imaging device 400, shown in FIG. 4A moves along the predetermined trajectory PT-PT' from a first position, for example position A, to capture the radiography images of the head 408 including the dental area of the patient. The imaging device 400 typically is an extraoral X-ray device having an X-ray source 402, which emits X-rays 406 that is allowed to pass through head including the dental area of the patient. Some X-rays 406 are absorbed by the teeth, which are typically shown in white color in the captured radiography images, and the remaining X-rays 406 are received at a receiver 404 (or detector or sensor) placed opposite the X-ray source 402. FIG. 4A shows that the X-ray 406 pass through one side of the jaw area without any interference caused by the opposing jaw area, also known as the 'opposing jaw effect'. Thus, the radiography images captured at position A of the imaging device 400 are not affected by any interference from the opposing jaw effect.

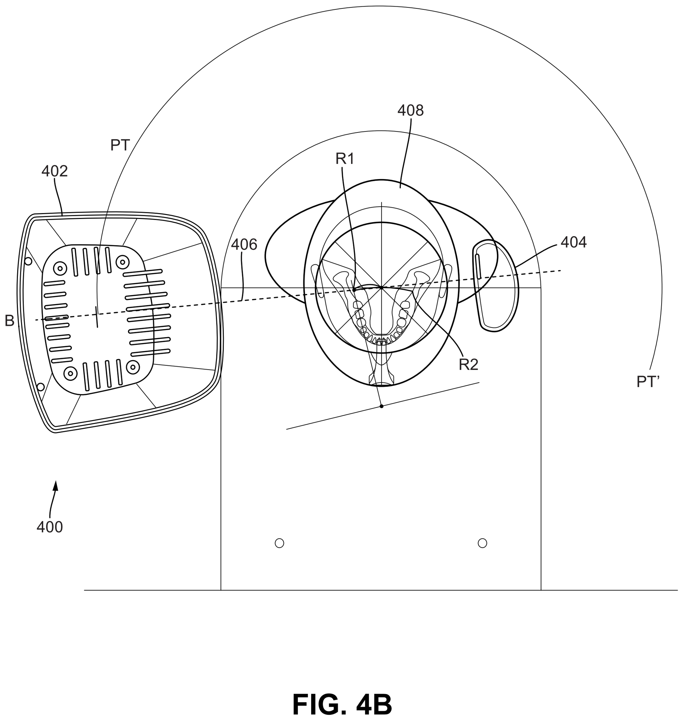

The imaging device 400 is configured to move along the predetermined trajectory PT-PT' of the x-ray source, emitting X-rays 406 from the X-ray source 402 at intervals, which may be set manually or automatically by the imaging device 400. The images may be acquired at a selected frame rate of the sensor/detector. FIG. 4B shows the imaging device 400 moved along the predefined trajectory PT-PT' to position B. At position B, the X-rays 406 emitted from the X-ray source 402 pass through both the left and right jaws, and can cause an opposing jaw effect, which can result in interference or overlays or shadows and thus reduce clarity of the captured radiography images. For example, along the predefined trajectory PT-PT', two rotation points R1 and R2 in the jaws (through which most of the X-rays 406 pass through) are typically associated with interference or overlays or shadows, thereby affecting the clarity of the captured radiography images.

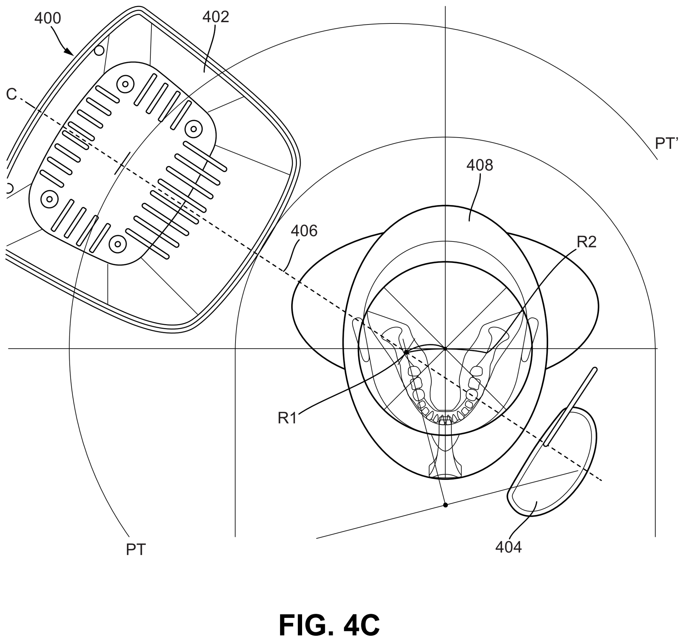

Referring to FIG. 4C, where the imaging device 400 is at position C, X-rays 406 pass through the sides of the head 408, through the rotation point R1 and the opposing jaw area and are received at the other end by the receiver 404. The radiography images captured at position C contain interference caused by the opposing jaw effects and the dental area in the radiography images may show overlays or shadows from opposing jaw in the X-ray path.

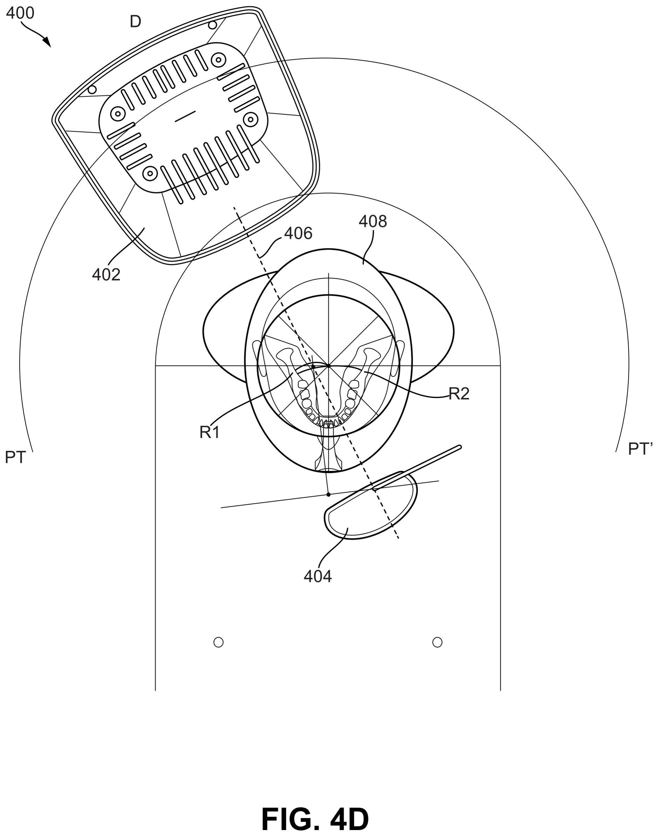

Referring to FIG. 4D, where the imaging device 400 is at position D, X-rays 406 do not pass through the rotation points R1, R2 and are less affected by the opposing jaw effects. The X-rays pass largely unobstructed through the dental area near the front of the face and is received at the other end by the receiver 404. The radiography images captured at position D are typically free from interference caused by the opposing jaw effect and the artifacts or structures such as cavity fillings in the dental area can be easily identified from the captured radiography images.

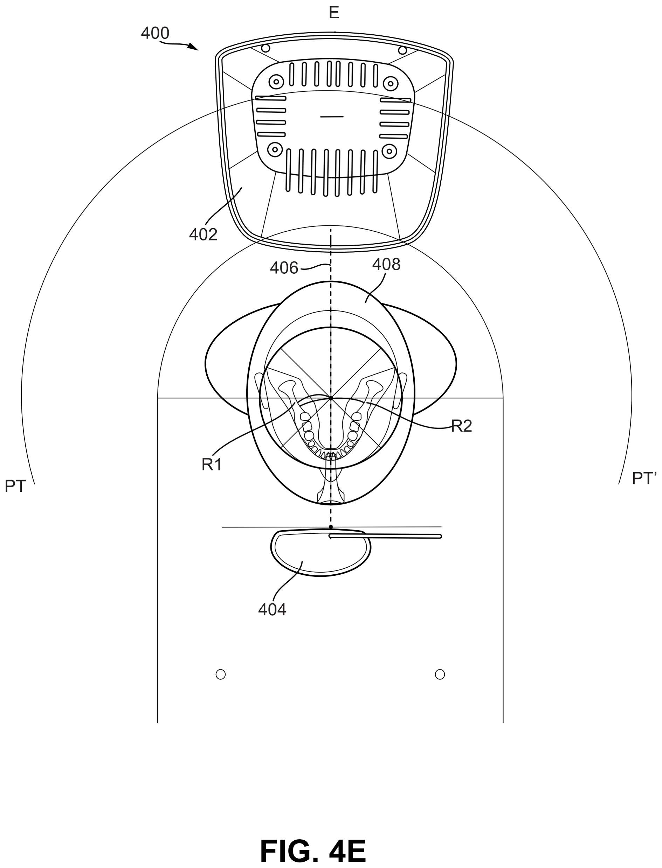

FIG. 4E shows the imaging device 400 moved along the predefined trajectory PT-PT' to position E, near the back of the head 408 of the patient, to capture the radiography images. At position E, the X-rays 406 emitted by the X-ray source 402 do not pass through the rotation points R1 or R2, thus avoiding opposing jaw interference. However, the spinal column of the patient may lie within the X-ray path and absorbs significant amount of X-rays, causing white shadows or overlays on the radiography images, which significantly affects the clarity of the radiography images.

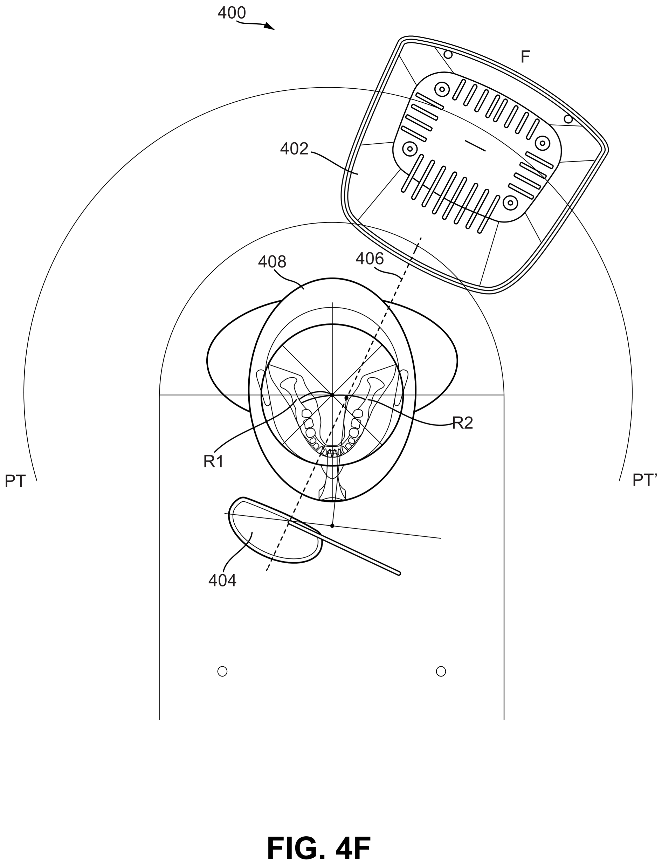

The imaging device 400 then moves to the right of the patient's head 408 along the predefined trajectory PT-PT' to reach position F as shown in FIG. 4F. At position F, the X-rays 406 emitted by the X-ray source 402 do not pass through the rotation points R1 or R2 and the resulting radiography images captured are not affected by the opposing jaw effect. However, as the imaging device 400 continues to move further right along the predefined trajectory PT-PT' beyond position F, the X-rays 406 emitted by the X-ray source 402 may pass through the rotation point R2, creating opposing jaw effect.

Once multiple radiography images of the dental area of the patient are captured by moving the x-ray source and x-ray-detector along the predetermined trajectory PT-PT' at a selected speed or frame rate, the method, in block 302, searches for 3D images or 3D models of the same body part of the same patient in the data source 108 or storage unit that communicates with the imaging device 400 over the network 102. In some instances, the data source 108 or storage unit is a cloud storage that contains 3D images or 3D models and associated data of the corresponding body part of multiple patients. In some other instances, the data source 108 or storage unit is a local storage in a hospital or medical facility and a network connection is not required to access the stored 3D images or 3D models.

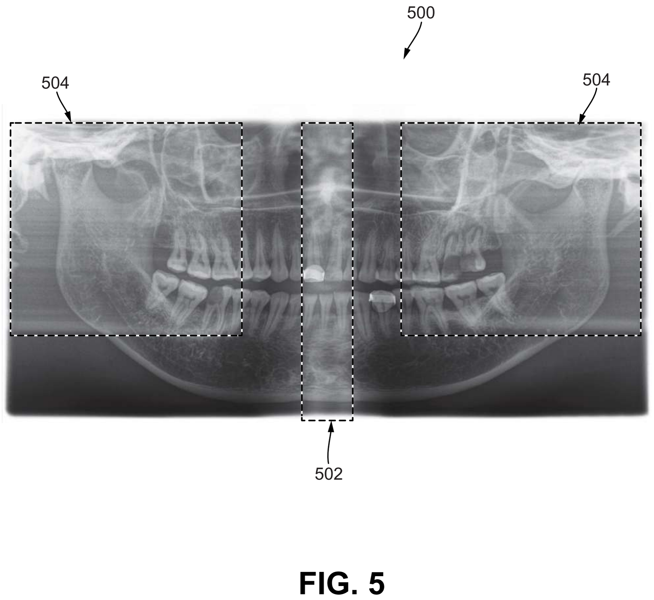

In block 304, the multiple radiography images captured by moving the imaging device 400 along the predetermined trajectory PT-PT' are combined using existing image processing methods to form a single panoramic radiography image with interferences or overlays present in the individual radiography images captured in block 300. FIG. 5 shows an example radiography image 500 with interference from the spinal column (spinal column interference 502) and from the opposing jaws (opposing jaw effects 504).

In some embodiments, the frame rate of the radiography images is selected such that significant overlaps occur between the adjacent radiography images. The image processing methods typically analyze the (3D) or geometric positions of each of the adjacent radiography images based on the respective time stamp and aligns them to form the panoramic radiography image. In some cases, the panoramic radiography image obtained by combining the individual radiography images may cause overlaps, which in turn affects the overall clarity of the panoramic radiography image. Hence, diagnosis based on the panoramic radiography image formed from the individual radiography images in block 304 is difficult and often not accurate.

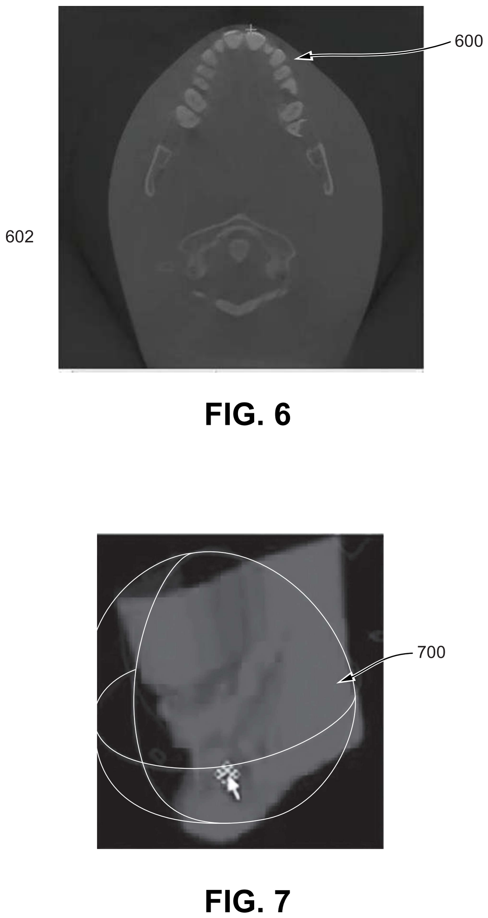

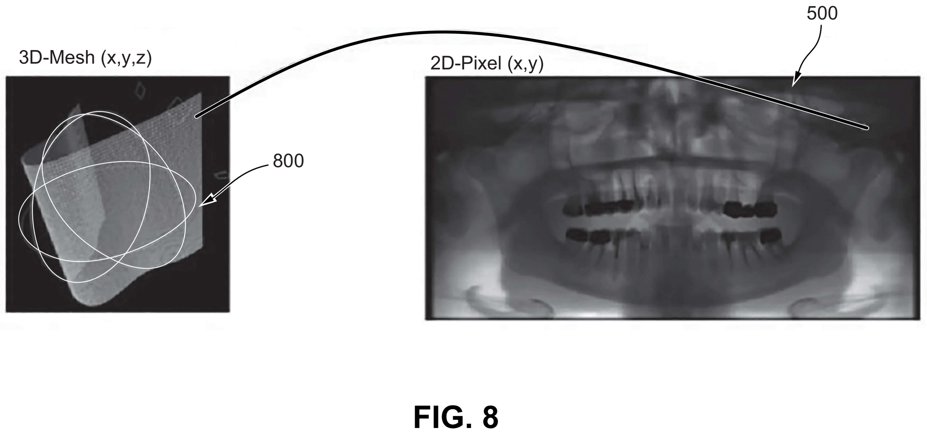

Typically, the dental images of patients are stored as cone beam computed tomography (CBCT) data or slices 600 as shown in FIG. 6, which provides a comprehensive 3D overview of the dental area of the patients. The slices 600 can be reconstructed to generate a 3D model. The 3D model or CBCT data (volume) is registered with the patient scan or panoramic image. The registration of the CBCT data (volume) with the patient scan is performed in block 306 and can be based on a 3D mesh 700 of the panoramic scan as shown in FIG. 7. Each point of the mesh may represent a specific pixel of the panoramic image 500 as shown in FIG. 8. The registration ensures that the patient's head has the same orientation (inclination, rotation, etc.) and size (resolution, scaling) within the CBCT data (volume) as within the actual panoramic image of the patient. In some embodiments, the 3D mesh 700 may be further contoured to the jawline or teeth for consistency with the panoramic image dimensions and orientation.

In other embodiments, the 3D mesh is a non-contoured 3D mesh 800. In an embodiment, the 3D model and 3D mesh 800 is placed in the same coordinate system as the panoramic radiography image 500, and a plurality of anatomical features in the 3D model are matched with corresponding features in the 3D mesh 700, 800. The alignment of the 3D model and 3D mesh 700, 800, ensures that the patient's head has the same orientation (inclination, rotation, etc.) and size in the CBCT data or 3D model as the actual panoramic radiography image of the patient.

As shown in FIG. 8, each point of the 3D mesh 800 is mapped to the corresponding 2D pixel in the panoramic radiography image 500. This can be formed in some cases using AI-based image processing methods.

In block 308, the method identifies one or more interference areas in the 3D model that are responsible for interference in the panoramic radiography image 500 based on the predetermined panoramic trajectory PT-PT'. In one embodiment, identifying the one or more interference areas in the 3D model includes detecting low-frequency artifacts such as tooth fillings from opposing jaw structures and background shadows from a spinal column or other anatomical features that block X-rays 406 generated by the X-ray source 402 while moving along the predetermined panoramic trajectory PT-PT'.

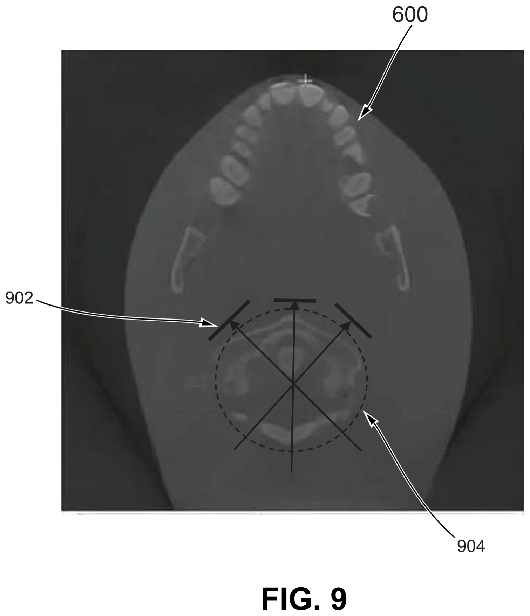

Once the interference areas in the 3D model are identified, block 310 generates a plurality of virtual panoramic projection images 902 (See FIG. 9) for areas 904 of the patient responsible for producing the identified one or more interference areas. In an embodiment, the plurality of virtual panoramic projection images is generated using the same projection geometry and a frame rate as used to form the panoramic radiography image 500.

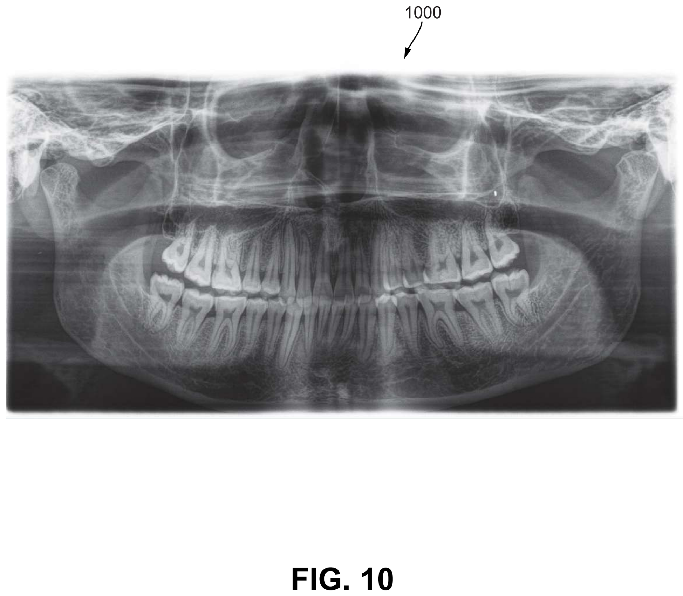

In block 312, the method utilizes information from the virtual panoramic projection images to remove the one or more interference areas from the plurality of radiography images or the panoramic radiography image 500 through an image processing method such as weighted subtraction. A new panoramic radiography image may therefore be formed (final radiographic panoramic image 1000 (see FIG. 10)), or the old panoramic image may be modified to generate the final radiographic panoramic image 1000, which is free or substantially free from interference caused by low-frequency interfering structures such as opposing jaw artifact, spinal shadows, and other anatomical features or structures that come in the X-ray path. The imaging device 400 performs the processing of the panoramic radiography image 500 in real-time or near real-time, providing clear panoramic radiography image 500 to medical practitioners for accurate diagnosis. The AI-based image processing methods help to identify different types of interferences such as those caused by tooth lesions or other interference and adjusts the detection process, accordingly, further enhancing clarity of the panoramic radiography image 500 generated in real-time with fewer structures in the background and/or superimposed interfering structures within the panoramic radiography image 500. The method works with existing imaging devices capable of capturing panoramic radiography images and can also leverage 2D imaging devices if CBCT data is available externally.

In an embodiment, removing the one or more interference areas from the radiography images or the panoramic radiography image 500 includes projecting the one or more interference areas from the 3D model to form the plurality of virtual panoramic projection images 902, modifying individual radiography images of the plurality of radiography images by removing the one or more interference areas from the individual radiography images based on information from the plurality of virtual panoramic projection images 902 and then reconstructing a final panoramic radiography image 1000 without the one or more interference areas based on the modified individual panoramic radiography images.

**In** an alternate embodiment, removing the one or more interference areas from the plurality of radiography images or the panoramic radiography image 500 includes projecting the one or more interference areas from the 3D model to form the plurality of virtual panoramic projection images 902, generating a separate panoramic radiography image of the identified interference areas using the plurality of virtual panoramic projection images and removing the one or more interference areas from the panoramic radiography image 500 using the separate panoramic radiography image.

### Conclusion

Any specific manifestations of these and other similar example processes are not intended to be limiting to the disclosure. Any suitable manifestation of these and other similar example processes can be selected within the scope of the illustrative embodiments.

Thus, a computer-implemented method, system or apparatus, and computer program product are provided in the illustrative embodiments for generating a panoramic radiography image with reduced interference and other related features, functions, or operations. Where an embodiment or a portion thereof is described with respect to a type of device, the computer-implemented method, system or apparatus, the computer program product, or a portion thereof, are adapted or configured for use with a suitable and comparable manifestation of that type of device.

Where an embodiment is described as implemented in an application, the delivery of the application in a Software as a Service (SaaS) model is contemplated within the scope of the illustrative embodiments. In a SaaS model, the capability of the application implementing an embodiment is provided to a user by executing the application in a cloud infrastructure. The user can access the application using a variety of client devices through a thin client interface such as a web browser, or other light-weight client-applications. The user does not manage or control the underlying cloud infrastructure including the network, servers, operating systems, or the storage of the cloud infrastructure. In some cases, the user may not even manage or control the capabilities of the SaaS application. In some other cases, the SaaS implementation of the application may permit a possible exception of limited user-specific application configuration settings.

The present disclosure may be a system, a method, and/or a computer program product at any possible technical detail level of integration. The computer program product may include a computer-readable storage medium (or media) having computer-readable program instructions thereon for causing a processor to carry out aspects of the present disclosure.

The computer-readable storage medium can be a tangible device that can retain and store instructions for use by an instruction execution device. The computer-readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer-readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer-readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer-readable program instructions described herein can be downloaded to respective computing/processing devices from a computer-readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer-readable program instructions from the network and forwards the computer-readable program instructions for storage in a computer-readable storage medium within the respective computing/processing device.

Computer-readable program instructions for carrying out operations of the present disclosure may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer-readable program instructions may execute entirely on a dedicated system or user's computer, partly on the user's computer or dedicated system as a stand-alone software package, partly on the user's computer and partly on a remote computer or entirely on the remote computer or server, etc. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer-readable program instructions by utilizing state information of the computer-readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to embodiments of the disclosure. It will be understood that each block of the flowchart illustrations and/or block diagrams, and combinations of blocks in the flowchart illustrations and/or block diagrams, can be implemented by computer-readable program instructions.

These computer-readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer-readable program instructions may also be stored in a computer-readable storage medium that can direct a computer, a programmable data processing apparatus, and/or other devices to function in a particular manner, such that the computer-readable storage medium having instructions stored therein comprises an article of manufacture including instructions which implement aspects of the function/act specified in the flowchart and/or block diagram block or blocks.

The computer-readable program instructions may also be loaded onto a computer, other programmable data processing apparatus, or other device to cause a series of operational steps to be performed on the computer, other programmable apparatus or other device to produce a computer-implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present disclosure. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

All features disclosed in the specification, including the claims, abstract, and drawings, and all the steps in any method or process disclosed, may be combined in any combination, except combinations where at least some of such features and/or steps are mutually exclusive. Each feature disclosed in the specification, including the claims, abstract, and drawings, can be replaced by alternative features serving the same, equivalent, or similar purpose, unless expressly stated otherwise.

## Claims

1. A method comprising:
capturing a plurality of radiography images of a body part of a patient along a predetermined panoramic trajectory using an imaging device;
retrieving at least one three-dimensional (3D) model of the body part of the patient from at least one data source;
forming a panoramic radiography image of the body part of the patient from the plurality of radiography images;
registering the 3D model with the panoramic radiography image;
identifying one or more interference areas in the 3D model that are responsible for interference in the panoramic radiography image;
generating a plurality of virtual panoramic projection images for the one or more interference areas using the 3D model, the plurality of virtual panoramic projection images is generated using a geometry and a frame rate as used to form the panoramic radiography image; and
removing the one or more interference areas from the plurality of radiography images or the panoramic radiography image based on the plurality of virtual panoramic projection images.

2. The method of claim 1, wherein retrieving the 3D model of the body part of the patient from at least one data source comprises:
receiving cone beam computed tomography (CBCT) data of the body part of the patient stored in the at least one data.

3. The method of any one of claims 1 or 2, wherein registering the 3D model with the panoramic radiography image includes aligning a plurality of anatomical features in the 3D model with corresponding features in the panoramic radiography image.

4. The method of any one of claims 1-3, wherein identifying the one or more interference areas includes detecting at least one of:
a plurality of low-frequency artifacts from opposing jaw structures; and
a plurality of background shadows from a spinal column or a plurality of anatomical features.

5. The method of any one of claims 1-4, wherein removing the one or more interference areas from the plurality of radiography images or the panoramic radiography image comprises:
projecting the one or more interference areas from the 3D model to generate the plurality of virtual panoramic projection images;
modifying individual radiography images of the plurality of radiography images by removing the one or more interference areas from the individual radiography images based on the plurality of virtual panoramic projection images; and
reconstructing the panoramic radiography image without the one or more interference areas based on the modified individual panoramic radiography images.

6. The method of any one of claims 1-4, wherein removing the one or more interference areas from the plurality of radiography images or the panoramic radiography image comprises:
projecting the one or more interference areas from the 3D model to generate the plurality of virtual panoramic projection images;
generating a separate panoramic radiography image of the identified interference areas using the plurality of virtual panoramic projection images; and
removing the one or more interference areas from the panoramic radiography image using the separate panoramic radiography image.

7. The method of any one of claims 1-6, wherein the body part is a head or dental region of the patient.

8. The method of any one of claims 1-7, wherein the removing is performed in real-time.

9. An imaging device comprising:
an x-ray source;
an x-ray detector;
a processor;
a storage unit in communication with the processor and is configured to receive and store a plurality of radiography images of a body part of a patient captured along a predetermined panoramic trajectory using the imaging device; and
a memory, in communication with the processor, with one or more computer program instructions stored on the memory, the computer program instructions, when executed by the processor, cause the imaging device to perform the method of any one of claims 1-8.

10. A non-transitory computer-readable medium having instructions, which when executed by a processor of an imaging device, cause the imaging device to perform the method of any one of claims 1-8.
